# EUROPEAN PATENT APPLICATION

(11) **EP 4 772 028 A1**
(43) Date of publication of application: **08.07.2026**
(21) Application number: 24860111.4
(22) Date of filing: 25.06.2024
(51) Int. Cl.: A24B 13/00, A24F 23/02, A61K 31/465, A61K 9/00, A24B 15/18, A24B 15/24, A24B 15/28, A24B 15/30

(54) **ORAL POUCH FILLER AND ORAL POUCH CONTAINING SAME**

(30) Priority: 31.08.2023 KR 20230115658
(71) Applicant: KT&G Corporation, Daedeok-gu Daejeon 34337 (KR)
(72) Inventor: KI, Sung Jong, Daejeon 34128 (KR); JEOUNG, Eunmi, Daejeon 34128 (KR); CHA, Sung Je, Daejeon 34128 (KR); SHIN, Jun Won, Daejeon 34128 (KR)
(74) Representative: Ter Meer Steinmeister & Partner
(86) International application number: PCT/KR2024/008746
(87) International publication number: WO 2025/048187

(57) **Abstract**

The present invention relates to an oral pouch filler containing an active substance, the oral pouch filler comprising: an excipient comprising cellulose and sugar alcohols; a binder, a pH adjuster; and a flavoring agent, wherein the sugar alcohols include sorbitol, and when the oral pouch filler is used, the release rate of the active substance is controlled such that the absorption rate of the active substance into the body of a user can be controlled.

## Description

### TECHNICAL FIELD

One or more embodiments relate to a filler for an oral pouch and an oral pouch including the filler.

### BACKGROUND ART

Oral pouches are pouches used in the user's mouth to allow the user to absorb active materials such as nicotine, and oral products in a form of pouches, such as snus, are manufactured and packaged in an appropriate form using granules containing active materials, including nicotine, using pouch packaging materials. Consumers may feel the satisfaction of the active materials for a predetermined period of time by placing the product in the form of a pouch between the gums and upper lip in the mouth. The elution characteristics of such active materials are the most essential characteristic of oral products and may be adjusted depending on the characteristics of a pouch material, but may be affected by a combination of fillers and a preparation method.

Therefore, research is required for more effective elution characteristics of active materials, including nicotine, from oral pouch fillers.

### DISCLOSURE OF THE INVENTION

### TECHNICAL GOALS

Embodiments provide a filler for an oral pouch with improved elution characteristics of an active material such as nicotine, and a filler for oral pouch with a specific type and addition ratio of sugar alcohol, that may delay release of the active material, limited to sugar alcohol among ingredients of a bulking agent included in the filler.

However, goals to be achieved are not limited to those described above, and other goals not mentioned above are clearly understood by one of ordinary skill in the art from the following description.

### TECHNICAL SOLUTIONS

According to an aspect, there is provided a filler for an oral pouch including an active material, an excipient including cellulose and sugar alcohol, a binder, a pH adjuster, and a flavoring agent, wherein the sugar alcohol includes sorbitol.

According to another aspect, there is provided an oral pouch including the filler for the oral pouch, and a packaging material that wraps the filler.

### EFFECTS OF THE INVENTION

When a filler for an oral pouch according to an embodiment is used, a release rate of an active material such as nicotine may be appropriately adjusted (specifically, nicotine release may be delayed), to adjust an absorption rate of the active substance into a user's body, thereby providing the user with satisfaction of smoking.

It should be understood that the effects of the present disclosure are not limited to the above-described effects, but are construed as including all effects that may be inferred from the configurations and features described in the following description or claims of the present disclosure.

### BRIEF DESCRIPTION OF THE DRAWINGS

These and/or other aspects, features, and advantages of the invention will become apparent and more readily appreciated from the following description of embodiments, taken in conjunction with the accompanying drawings of which:

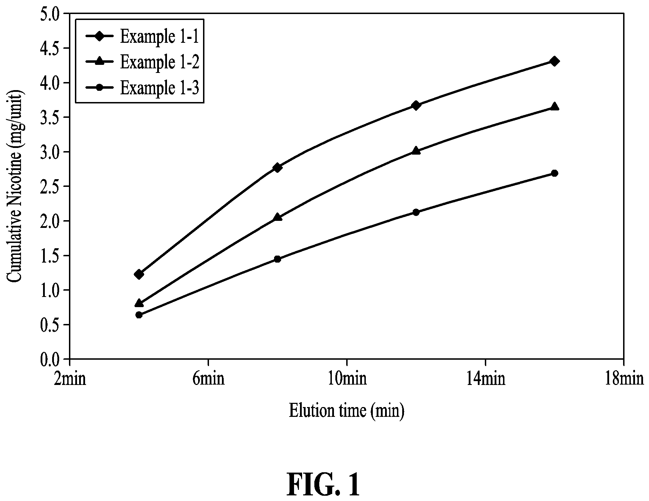

FIG. 1 is a diagram illustrating a graph of a cumulative nicotine elution over time for each oral pouch according to an embodiment;

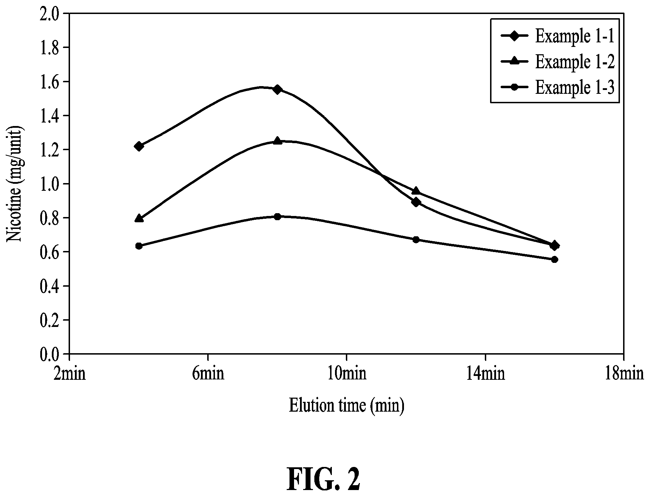

FIG. 2 is a diagram illustrating a graph of nicotine elution over time for each oral pouch according to an embodiment; and
FIG. 3 illustrates an elution rate according to a content of microcrystalline cellulose (MCC) of an oral pouch according to an embodiment.

### BEST MODE FOR CARRYING OUT THE INVENTION

Hereinafter, embodiments will be described in detail with reference to the accompanying drawings. However, various alterations and modifications may be made to the embodiments. Here, the embodiments are not construed as limited to the disclosure. The embodiments should be understood to include all changes, equivalents, and replacements within the idea and the technical scope of the disclosure.

The terminology used herein is for the purpose of describing particular embodiments only and is not to be limiting of the embodiments. The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like components and a repeated description related thereto will be omitted. In the description of embodiments, detailed description of well-known related structures or functions will be omitted when it is deemed that such description will cause ambiguous interpretation of the present disclosure.

In addition, the terms first, second, A, B, (a), and (b) may be used to describe constituent elements of the embodiments. These terms are used only for the purpose of discriminating one component from another component, and the nature, the sequences, or the orders of the components are not limited by the terms.

A component, which has the same common function as a component included in any one embodiment, will be described by using the same name in other embodiments. Unless otherwise mentioned, the descriptions of an embodiment may be applicable to other embodiments and thus, repeated descriptions will be omitted for conciseness.

It will be understood that when a certain part "includes" a certain component, the part does not exclude another component but may further include another component.

According to an embodiment, there is provided a filler for an oral pouch including an active material, an excipient including cellulose and sugar alcohol, a binder, a pH adjuster, and a flavoring agent, wherein the sugar alcohol includes sorbitol.

The filler for the oral pouch according to an embodiment may realize suitable elution characteristics of an active material existing in a pouch as the filler has the configuration described above.

Effective elution characteristics of a product in a pouch form refers to that, from the consumer's point of view, a quick recognition or satisfaction is required due to the elution of the active material during use, and at the same time, all applied active material must be eluted within a predetermined period of time. For example, for a product in a pouch form with a small size, it is desirable that the size is 30 mm × 12 mm and weighs 0.3 to 0.5 g, and the active material is eluted out during a usage time of approximately 20 minutes.

The filler for the oral pouch according to an embodiment may include an excipient, a binder, a pH adjuster, and a flavoring agent together with the active material.

The active material is a material that may be absorbed through the oral mucosa or gums, and may include, specifically, nicotine, vitamin, caffeine, amino acid, a salt or a derivative thereof, and desirably, nicotine and a nicotine salt. The nicotine may be synthetic nicotine and/or a nicotine extract from tobacco plant, and the type of nicotine salt is not limited, and may include nicotine salt benzoate, nicotine salt tartrate, nicotine salt malate, nicotine salt salicylate, and the like.

Particularly, when a nicotine derivative or the like is applied as the active material, the pH adjuster may be applied to generate free nicotine by providing a basic environment during product use, and a pH of the filler for the oral pouch may be adjusted specifically to 7 to 9 using the pH adjuster. Specific examples of the pH adjuster may include metal hydroxide such as sodium hydroxide and potassium hydroxide, metal carbonate such as sodium carbonate and potassium carbonate, sodium bicarbonate, sodium sesquicarbonate (Na₃H(CO₃)₂), and the like. In particular, the pH adjuster is preferably added so that the pH of the filler is at the level of 8.3 to 8.5 for effective nicotine absorption. Thus, it is desirable that the pH adjuster includes sodium bicarbonate and sodium carbonate at a ratio of 7:3 to 6:4.

The excipient may include cellulose, sugar alcohol, a non-tobacco plant material, and the like, and in an embodiment, the excipient includes cellulose and sugar alcohol. The cellulose may function as a carrier for an active material such as nicotine, and also function as a diluent with a high water holding capacity to promote the wetting of a mixture in a powder form through swelling and wicking and quickly release saliva and nicotine. Also, the cellulose may provide usability. When the cellulose is not added, the filler for the oral pouch may come out in a dissolved form.

The cellulose may include microcrystalline cellulose (MCC), cellulose, a cellulose derivative, and the like, and specific examples thereof may include MCC, methylcellulose, hydroxypropyl cellulose (HPC), hydroxypropylmethyl cellulose (HPMC), hydroxyethyl cellulose, carboxymethyl cellulose, and the like.

More specifically, the cellulose may include MCC, and a content of the MCC may be 1 to 2 times a content of the active material such as nicotine, and may be included in an amount of 5 to 20 wt% with respect to total solid contents of the filler. When the content of the cellulose is below the numerical range described above, the cellulose may not properly function as a carrier for nicotine, or the like, and the filler for the oral pouch may dissolve quickly. On the other hand, when the content of the cellulose exceeds the numerical range described above, a nicotine elution rate may be excessively delayed due to a water-insoluble characteristic of cellulose.

Meanwhile, the sugar alcohol is highly soluble in saliva and loosely binds to a solvent than the cellulose. Accordingly, the sugar alcohol may control nicotine release within the time of use, suppress a bitter taste, and provide a cooling effect when dissolved in the mouth. In addition, the sugar alcohol may promote nucleation and granule growth in a high-shear wet granulation process of the filler for the oral pouch.

A content of the sugar alcohol may be 50 to 70 wt%, desirably, 60 to 70 wt% with respect to a weight of the total solid contents of the filler for the oral pouch. When the content of the sugar alcohol is below the numerical range described above, nuclei of particles of the filler for the oral pouch may not be sufficiently formed. Meanwhile, when the content of the sugar alcohol exceeds the numerical range described above, severe agglomeration or crushing of a powder may occur, making it difficult to prepare the filler for the oral pouch.

In an embodiment, considering the effect of delaying the nicotine release rate, it may be said that the sugar alcohol desirably includes sorbitol, and the sorbitol is more desirably included in an amount of 50 wt% or more with respect to a total content of the sugar alcohol from the effective point of view. In addition, in addition to the sorbitol, the sugar alcohol may further include isomalt, erythritol, arabitol, ribitol, maltitol, dulcitol, iditol, mannitol, xylitol, lactitol, and the like. Furthermore, examples of the non-tobacco plant material are not greatly limited and may include a coconut shell, a vegetable fiber, tea, herbs, coffee, fruits, and the like.

The binder may include one or more selected from a group consisting of HPC, HPMC, polyvinylpyrrolidone (PVP), sodium alginate, xanthan gum, guar gum, gellan gum, and gum arabic, and the amount used thereof may be 10 wt% or less, desirably, 5 to 8 wt% with respect to the filler.

The flavoring agent may also be referred to as a "flavoring material", and refers to any aromatic substance that may change sensory properties associated with an oral product. The flavoring agent may be a natural flavoring agent or a synthetic flavoring agent, and specific types thereof may include vanilla, coffee, chocolate/cocoa, cream, mint, spearmint, menthol, peppermint, winter green, eucalyptus, lavender, cardamone, nutmeg, cinnamon, clove, cascarilla, Santalum album, honey, ginger, anise, sage, licorice, lemon, orange, apple, peach, lime, cherry, strawberry, and the like, but are not limited thereto.

In addition, the filler for the oral pouch according to an embodiment may further include a sweetener, a humectant, an antioxidant, a preservative, and the like depending on the purpose and need of use, in addition to the composition described above.

The sweetener may be a natural sweetener or an artificial sweetener, and specific types thereof may include fructose, sucrose, glucose, maltose, mannose, galactose, lactose, stevia, honey, sucralose, isomaltose, maltodextrin, saccharin, aspartame, and the like, but are not limited thereto.

For example, the humectant may include at least one or more of glycerin and propylene glycol.

According to an embodiment, an oral pouch including the filler for the oral pouch described in detail above, and a packaging material that wraps the filler may be provided.

The packaging material for the oral pouch is a material that is safe for the human body, may hold saliva well, has a predetermined strength to prevent tearing, and has a sealing property, and a material of the packaging material for the oral pouch may be a nonwoven-type or woven-type material, desirably a nonwoven-type material. Unlike the woven type material, the nonwoven type material has tangled fibers, and thus, there may be no vertical or horizontal direction, and cut edges may not unravel.

The packaging material for the oral pouch may specifically include cellulose fibers and thermoplastic material fibers.

At this time, the cellulose fibers may include not only cellulose but also a cellulose-based fiber, and the cellulose-based fiber may include at least one or more of a natural cellulose-based fiber or an artificial cellulose-based fiber.

In addition, the natural cellulose-based fiber may include at least one or more selected from a group consisting of a seed hair fiber, a bast fiber, a leaf fiber, and a wood fiber (pulp). The seed hair fiber may include, for example, cotton, bombax cotton, kapok, and the like. The bast fiber may include, for example, hemp, flax, jute, ramie grass, mulberry tree, and edgeworthia chrysantha, and the like. The leaf fiber may include, for example, Manila hemp, New Zealand hemp, and the like. The wood fiber (pulp) may include at least one or more of softwood pulp or hardwood pulp, depending on the type and characteristics of the tree, and examples thereof may include a softwood fiber, a hardwood fiber, and the like.

The artificial cellulose-based fiber may include at least one or more of a regenerated cellulose fiber or a semi-synthetic fiber. The regenerated cellulose fiber may include, for example, viscose, rayon, forty acid, and the like. The semi-synthetic fiber may, for example, include an acetate fiber. The rayon may include not only rayon but also a rayon-based fiber that compensates for the shortcomings of rayon. The rayon-based fiber may include, for example, Tencel, lyocell, modal, and the like.

Meanwhile, the thermoplastic material fiber may include at least one or more selected from a group consisting of nylon, polyethylene, polylactic acid, polypropylene, polyethersulfone, and polyethylene terephthalate. By including the thermoplastic material fiber, the packaging material for the oral pouch may be sealed by heat.

Hereinafter, the present disclosure will be described in more detail with reference to examples, however, the present disclosure is not limited to the examples below.

### 1. Example 1: Experimental evaluation according to type and content of sugar alcohol

### (1) Preparation Example 1-1: Preparation of filler for oral pouch

Example 1-1: After first mixing the nicotine, the excipient (MCC, maltitol), the binder, the pH adjuster, and the flavoring agent, a solvent, that is water alone, water + alcohol, or alcohol alone, was sprayed, and a granulation process was performed to prepare a filler for an oral pouch. At this time, the contents of MCC and maltitol were 25 wt% and 50 wt%, respectively, with respect to a weight of the total solid contents of the filler for the oral pouch.

Example 1-2: A filler was prepared in the same manner as in Example 1-1, except that the excipient was composed of 25 wt% of MCC, 25 wt% of maltitol, and 25 wt% of sorbitol with respect to the total solid contents.

Example 1-3: A filler was prepared in the same manner as in Example 1-1, except that the excipient was composed of 25 wt% of MCC and 50 wt% of sorbitol with respect to the total solid contents.

### (2) Experimental Example 1-1: Analysis of physical properties of oral pouch

For the pouches of Examples 1-1 to 1-3 prepared in Preparation Example 1-1, a total nicotine content, a pH, an average particle size, and a moisture content were analyzed and the results thereof were shown in Table 1 below.

**[Table 1]**

| Classification | Total Nic. (mg/pouch) | pH | Average particle size (µm) | Moisture (%) |
|---|---|---|---|---|
| Example 1 | 5.58 | 8.32 | 232 | 2.88 |
| Example 2 | 5.65 | 8.40 | 239 | 1.71 |
| Example 3 | 5.88 | 8.32 | 210 | 1.39 |

### (3) Experimental Example 1-2: Evaluation of nicotine elution characteristics

For the oral pouches of Examples 1-1 to 1-3 prepared in Preparation Example 1-1, an average nicotine amount and a cumulative nicotine amount by time period were measured. The results thereof were shown in Table 2 below.

**[Table 2]**

| Product name | Time | Average amount of nicotine | Cumulative amount of nicotine (mg/unit) |
|---|---|---|---|
| Example 1-1 | 4min | 1.223 | 1.223 |
| | 8min | 1.556 | 2.779 |
| | 12min | 0.896 | 3.675 |
| | 16min | 0.642 | 4.318 |
| Example 1-2 | 4min | 0.798 | 0.798 |
| | 8min | 1.250 | 2.047 |
| | 12min | 0.957 | 3.004 |
| | 16min | 0.640 | 3.644 |
| Example 1-3 | 4min | 0.637 | 0.637 |
| | 8min | 0.809 | 1.446 |
| | 12min | 0.676 | 2.122 |
| | 16min | 0.561 | 2.683 |

### (4) Experimental Example 1-3: Sensory evaluation of oral pouch

For the oral pouches of Examples 1-1 to 1-3 prepared in Preparation Example 1-1, a sensory evaluation was conducted on our panel by using the oral pouches for 30 minutes and then resting for 1 hour, and the results thereof were shown in Table 3 below.

**[Table 3]**

| Classification | Example 1-1 | Example 1-2 | Example 1-3 |
|---|---|---|---|
| Time when initial gum irritation starts ( seconds) | 30 seconds | 50 seconds | 60 seconds |
| Intensity of initial gum irritation <7 points> | 3.5 | 3.0 | 2.5 |
| Time to recognize Nicotine satisfaction (minutes) | 3 minutes | 4 minutes | 4 minutes |
| Nicotine Satisfaction <7 points> | 5.0 | 4.5 | 4.0 |
| Nicotine Satisfaction Duration (minutes) | 20 minutes | 20 minutes | 20 minutes |

### 2. Example 2: Nicotine elution rate according to content of MCC

### (1) Preparation Example 2-1: Preparation of filler for oral pouch

Example 2-1: After first mixing nicotine, MCC, maltitol, the binder, the pH adjuster, and the flavoring agent, a solvent, that is water alone, water + alcohol, or alcohol alone, was sprayed, and a granulation process was performed to prepare a filler for an oral pouch. At this time, the binder was HPC, and a content of the MCC was 20 wt% with respect to a weight of the total solid contents of the filler for the oral pouch.

Example 2-2: A filler for an oral pouch was prepared in the same manner as in Example 2-1, except that the content of the MCC was 25 wt%.

Comparative Example 2-1: A filler for an oral pouch was prepared in the same manner as in Example 2-1, except that the content of the MCC was 40 wt%.

Comparative Example 2-2: A filler for an oral pouch was prepared in the same manner as in Example 2-1, except that the content of the MCC was 55 wt%.

### (2) Experimental Example 2-1: Evaluation of nicotine elution characteristic

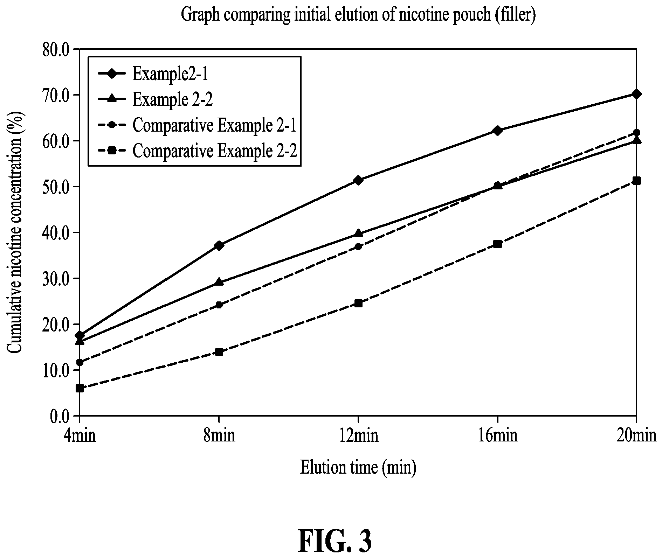

The nicotine elution rates of Examples 2-1 and 2-2 and Comparative Examples 2-1 and 2-2 were analyzed. The nicotine elution rate was evaluated by measuring a content of nicotine released after leaving the examples and comparative examples in 1 liter of a phosphate buffer maintained at a temperature of 37°C and a pH of 7.4 for 1 minute using a USP paddle device. The results thereof were shown in FIG. 3 and Table 4.

**[Table 4]**

| | Example 2-1 | Example 2-2 | Comparative Example 2-1 | Comparative Example 2-2 |
|---|---|---|---|---|
| MCC content (wt%) | 20 | 25 | 40 | 55 |
| Elution rate after first 4 minutes (%) | 17.5 | 16.1 | 11.7 | 6.1 |
| Elution rate after 20 minutes (%) | 70.1 | 59.9 | 61.7 | 51.2 |

In Example 2-1, up to 90% of the content of nicotine added was released after the first 1 minute, and the elution rate after the first 4 minutes was 17.5%, showing a highest elution rate. In addition, Example 2-2 showed a second highest result with the elution rate of 16.1% after the first 4 minutes. However, as the content of the MCC increased, the elution rate tended to decrease rapidly after the first 4 minutes due to the water-insoluble characteristic of the MCC (Comparative Examples 2-1 and 2-2).

### 3. Example 3: Nicotine elution rate according to addition of sugar alcohol

### (1) Preparation Example 3-1: Preparation of filler for oral pouch

Example 3-1: A filler for an oral pouch was prepared similarly to Example 2-1. At this time, a content of the maltitol was 70 wt% with respect to the weight of the total solid contents of the filler for the oral pouch, and a content of the MCC was 5 wt% with respect to the weight of the total solid contents.

Example 3-2: A filler for an oral pouch was prepared in the same manner as in Example 3-1, except that the content of the maltitol was 55 wt% with respect to the weight of the total solid contents, and accordingly, the content of the MCC was 20 wt% with respect to the weight of the total solid contents.

### (2) Experimental Example 3-1: Evaluation of nicotine elution characteristics

The nicotine elution rates of Examples 3-1 and 3-2 were analyzed. The nicotine elution rate was evaluated in the same manner as in the experimental example described above.

As a result, Example 3-1 showed the nicotine elution rate of 20% after the first 4 minutes and the nicotine elution rate of 80% after 20 minutes, and Example 3-2 showed the nicotine elution rate of 14% after the first 4 minutes and the nicotine elution rate of 51.6% after 20 minutes. This is determined to be due to the fact that, as the content of the MCC increases, the elution rate decreases after the first 4 minutes and after 20 minutes due to the water-insoluble characteristic of the MCC.

### 4. Example 4: Distribution of particle size and nicotine elution rate according to type of binder

### (1) Preparation Example 4-1: Preparation of filler for oral pouch

Example 4-1: After first mixing nicotine, MCC, maltitol, the binder, the pH adjuster, and the flavoring agent, a solvent, that is water alone, water + alcohol, or alcohol alone, was sprayed, and a granulation process was performed to prepare a filler for an oral pouch.

The binder was HPC, and a content of the binder was 8 wt% with respect to the total weight of the filler.

Example 4-2: A filler for an oral pouch was prepared in the same manner as in Example 4-1, except that povidone K-25 was used as the binder.

Example 4-3: A filler for an oral pouch was prepared in the same manner as in Example 4-1, except that low-substituted HPC was used as the binder.

### (2) Experimental Example 4-1: Evaluation of nicotine elution characteristic

The nicotine elution rates of Example 3-1 and Examples 4-1 to 4-3 were analyzed. The nicotine elution rate was evaluated in the same manner as in the experimental examples described above. The results thereof were shown in Table 5 below.

**[Table 5]**

| Classification | | Example 3-1 | Example 4-1 | Example 4-2 | Example 4-3 |
|---|---|---|---|---|---|
| Elution rate after first 4 minutes (%) | | 20 | 17.5 | 15.3 | 13.8 |
| Elution rate after 20 minutes (%) | | 80 | 70.1 | 61.9 | 55.3 |
| Distribution of particle size (%) | Size of 200 µm or more | 70 | 66.7 | 50.5 | 61.8 |
| | Size of 50 µm or less | None | None | 2.2 | 1.6 |

In Examples 3-1 and 4-1, 66.7% or more of particles of the filler for the oral pouch having a size of 200 µm or more were distributed, and there were no particles of the filler for the oral pouch having a size of 50 µm or less. Examples 3-1 and 4-1 showed higher nicotine elution rates not only in the initial stage but also after 20 minutes, compared to Examples 4-2 and 4-3.

### 5. Example 5: Free-base nicotine generation rate depending on pH range and proportion of pH adjuster

### (1) Preparation Example 5-1: Preparation of filler for oral pouch

Example 5-1: A filler for an oral pouch contained the pH adjuster, and sodium bicarbonate and sodium carbonate were used as the pH adjuster at a ratio of 6:4.

Example 5-2: A filler for an oral pouch was prepared in the same manner as in Example 5-1, except that sodium bicarbonate and sodium carbonate were used as the pH adjuster at a ratio of 7:3.

Comparative Example 5-1: A filler for an oral pouch was prepared in the same manner as in Example 5-1, except that sodium bicarbonate and sodium carbonate were used as the pH adjuster at a ratio of 8:2.

### (2) Experimental Example 5-1: Evaluation of nicotine elution characteristic

The pH and the free-base nicotine generation rates of the fillers for the oral pouch of Examples 5-1 and 5-2 and Comparative Example 5-1 were investigated, and the results thereof were shown in Table 6.

**[Table 6]**

| Classification | pH | Free-base nicotine generation rate (%) |
|---|---|---|
| Example 5-1 | 8.8 | 85% to 90% |
| Example 5-2 | 8.4 | 76% |
| Comparative Example 5-1 | 7.6 | 24% |

Unlike Comparative Example 5-1, Examples 5-1 and 5-2 showed results in which the pH of saliva was 8.8 and 8.4, respectively, and the free-base nicotine generation rate was 76% or more. When saliva with the pH of 6.5 to 7.7 is introduced, the pH becomes 8.02 or more, and thus, it is confirmed that more free-base nicotine is produced.

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order, and/or if components in a described system, architecture, device, or circuit are combined in a different manner, and/or replaced or supplemented by other components or their equivalents.

Therefore, other implementations, other embodiments, and equivalents to the claims are also within the scope of the following claims.

## Claims

1. A filler for an oral pouch comprising:
an active material;
an excipient comprising cellulose and sugar alcohol;
a binder;
a pH adjuster; and
a flavoring agent,
wherein the sugar alcohol comprises sorbitol.

2. The filler of claim 1, wherein the sorbitol is included in an amount of 50 wt% or more with respect to a total amount of sugar alcohol.

3. The filler of claim 1, wherein the active material comprises one or more selected from a group consisting of nicotine, vitamin, caffeine, salt thereof, and a derivative thereof.

4. The filler of claim 1, wherein
the cellulose comprises one or more selected from a group consisting of microcrystalline cellulose (MCC), methyl cellulose, hydroxypropyl cellulose (HPC), hydroxypropylmethyl cellulose (HPMC), hydroxyethyl cellulose, and carboxymethyl cellulose, and
the sugar alcohol comprises one or more selected from a group consisting of isomalt, erythritol, arabitol, ribitol, maltitol, dulcitol, iditol, mannitol, xylitol, and lactitol, in addition to sorbitol.

5. The filler of claim 1, wherein
the cellulose comprises MCC, and
a content of the MCC is 1 to 2 times a content of nicotine, and 5 to 20 wt% with respect to a weight of total solid contents of the filler for the oral pouch.

6. The filler of claim 1, wherein the binder comprises one or more selected from a group consisting of HPC, HPMC, polyvinyl pyrrolidone (PVP), sodium alginate, xanthan gum, guar gum, gellan gum, and gum arabic.

7. The filler of claim 1, wherein
the binder comprises HPC, and
a content of the HPC is 5 to 8 wt% with respect to a content of the filler.

8. The filler of claim 1, wherein the pH adjuster comprises one or more selected from a group sodium hydroxide, potassium hydroxide, sodium carbonate, potassium carbonate, sodium bicarbonate, and sodium sesquicarbonate (Na₃H(CO₃)₂).

9. The filler of claim 8, wherein
the pH adjuster comprises sodium bicarbonate and sodium carbonate,
the bicarbonate and sodium carbonate are included at a weight ratio of 7:3 to 6:4, and
a pH of the filler is 8.3 to 8.5.

10. The filler of claim 1, wherein the flavoring agent comprises one or more selected from a group consisting of vanilla, coffee, chocolate/cocoa, cream, mint, spearmint, menthol, peppermint, winter green, eucalyptus, lavender, cardamone, nutmeg, cinnamon, clove, cascarilla, Santalum album, honey, ginger, anise, sage, licorice, lemon, orange, apple, peach, lime, cherry, and strawberry.

11. The filler of claim 1, further comprising
one or more of a sweetener, a humectant, an antioxidant, and a preservative.

12. An oral pouch comprising:
the filler for the oral pouch of claim 1; and
a packaging material that wraps the filler.
